(19) Europäisches Patentamt
European Patent Office
Office européen des brevets

(11) Publication number: **0 074 768**
**A2**

# (12) EUROPEAN PATENT APPLICATION

(21) Application number: 82304642.0

(22) Date of filing: 03.09.82

(51) Int. Cl.³: **C 07 D 213/74**
C 07 D 401/04, A 61 K 31/395

(30) Priority: 03.09.81 GB 8126754

(43) Date of publication of application:
23.03.83 Bulletin 83/12

(84) Designated Contracting States:
AT BE CH FR LI SE

(71) Applicant: RECORDATI S.A.
Corso S. Gottardo 54
CH-6830 Chiasso(CH)

(72) Inventor: Nardi, Dante
Via T. Gulli 47
Milan(IT)

(72) Inventor: Tajana, Alberto
Via Omboni 3
Milan(IT)

(72) Inventor: Cazzulani, Pietro
Via F.-Cavalotti 112
Casal Pusterlengo Milan(IT)

(72) Inventor: Graziani, Gabriele
Via Dei Platini8
Arese Milan(IT)

(72) Inventor: Catto, Alberto
Viale Lunigiana 16
Milan(IT)

(74) Representative: SERJEANTS
25, The Crescent
Leicester, LE1 6RX(GB)

## (54) Alkanoylanilides.

(57) ω-[4-(2-pyridyl)-piperazino]-alkanoylanilide derivatives of the general formula I are provided.

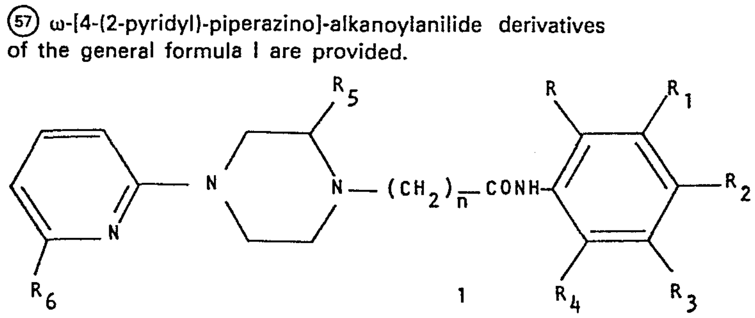

1

In the formula,

$n$ = 1, 2 or 3,

each of R, $R_1$, $R_2$, $R_3$ and $R_4$ is independently H, halogen, alkyl, alkoxy, OH, $NH_2$, $CF_3$, CN, alkylthio, 1-hydroxyalkyl, alkanoyl, $NH_2CO$, $NH_2SO_2$, alkoxycarbonyl, alkylsulphonyl, benzyloxy, alkanoylamino, $NH_2CONH$, 3-phenylureido, 3-alkylureido or alkoxyoxalylamino,

$R_5$ = H or alkyl,

$R_6$ = H or alkoxy.

These compounds and their pharmaceutically acceptable salts have antianaphylactic, antibronchospastic, antihistaminic, sedative, analgesic, antiserotonic and blood-pressure-lowering effects. Their preparation and pharmaceutical compositions containing them are disclosed.

EP 0 074 768 A2

Croydon Printing Company Ltd.

TITLE

Alkanoylanilides

DESCRIPTION

The invention relates to ω-/4-(2-pyridyl)-piperazino/-alkanoylanilide derivatives and pharmaceutically acceptable salts thereof, to processes for their production and to pharmaceutical compositions containing them.

The invention provides ω-/4-(2-pyridyl)-piperazino/-alkanoylanilide derivatives of the general formula I

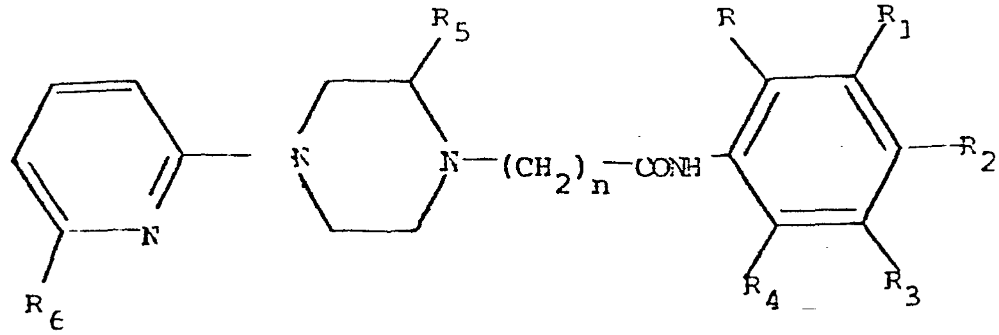

I

wherein n is 1,2, or 3, each of $R,R_1,R_2,R_3$ and $R_4$ independently represents a hydrogen or halogen atom, or an alkyl, alkoxy, hydroxy, nitro, amino, trifluoromethyl, cyano, alkylthio, 1-hydroxyalkyl, alkanoyl, carbamoyl, sulphamoyl, alkoxycarbonyl, alkylsulphonyl, benzyloxy, alkanoylamino, ureido, 3-phenylureido, 3-alkylureido or alkoxyoxalylamino group, $R_5$ represents a hydrogen atom or an alkyl group and $R_6$ represents a hydrogen atom or an alkoxy group, and further provides pharmaceutically acceptable acid addition salts of such ω-[4-(2-pyridyl)-piperazino]-alkanoylanilide derivatives. The terms alkyl and alkoxy as used above, whether alone or in combination, refer to such groups having from 1 to 4 carbon atoms; the term alkanoyl as used above, whether alone or in combination, refers to such a group having from 2 to 4 carbon atoms.

The compounds of the general formula I wherein n; $R_5$ and $R_6$ are as above defined and each of $R,R_1,R_2,R_3$ and $R_4$ independently represents a hydrogen or halogen atom, or an alkyl, alkoxy, hydroxy, nitro, trifluoromethyl, cyano, alkylthio, 1-hydroxyalkyl, alkanoyl, carbamoyl, sulphamoyl, alkoxycarbonyl, alkylsulphonyl or benzyloxy group may be prepared by a process comprising reacting a 4-(2-pyridyl)-piperazine of the general formula II

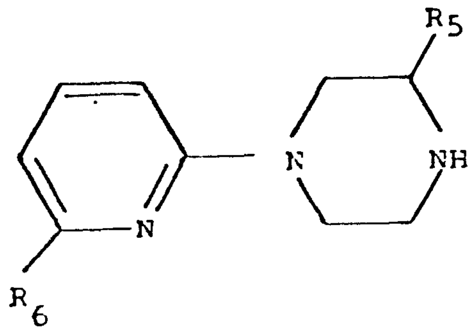

II

wherein $R_5$ and $R_6$ as above defined with an ω-halo-alkanoylanilide of the general formula III

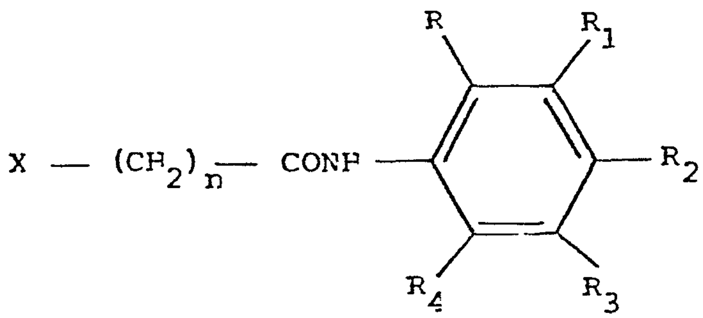

III

wherein X represents a halogen atom, n is as above defined and R, $R_1$, $R_2$, $R_3$ and $R_4$ are as defined in this sentence. The reaction is preferably carried out in the presence of an organic or inorganic base, such as triethylamine or alkali metal bicarbonate, and a solvent, such as an alkanol or a ketone having up to 4 carbon atoms, at the reflux temperature of the solvent.

The compounds of the general formula I wherein n is 2, $R_5$ and $R_6$ are as defined above and R, $R_1$, $R_2$, $R_3$ and $R_4$ are as defined in the last preceding paragraph may alternatively be prepared by reacting a 4-(2-pyridyl)-piperazine of

the general formula II as above defined with an acryloylanilide of the general formula IV

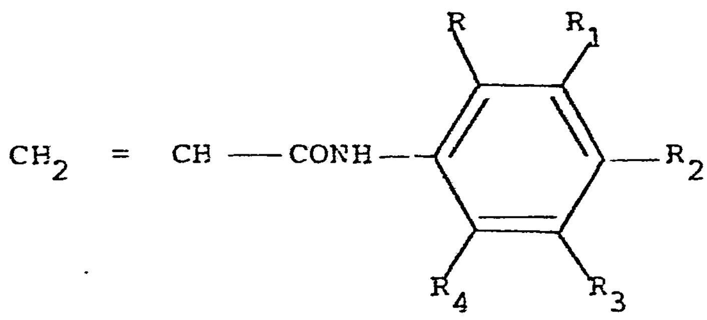

IV

wherein R, $R_1$, $R_2$, $R_3$ and $R_4$ are as defined in the last preceding paragraph. This reaction is preferably performed in a solvent, such as dimethylsulphoxide, chloroform or an aromatic hydrocarbon, for example toluene or xylene.

The compounds of the general formula I wherein any of R, $R_1$, $R_2$, $R_3$ and $R_4$ represents an amino group may be prepared by reduction of the corresponding nitro derivative, prepared by one of the afore described processes, the reduction being effected with hydrogen in the presence of a palladium-on-carbon catalyst and a solvent. These amino derivatives themselves are starting materials for the preparation of the corresponding compounds of the general formula I wherein any of R, $R_1$, $R_2$, $R_3$ and $R_4$ represents a ureido, 3-phenylureido, 3-alkylureido,

alkanoylamino or alkoxyoxalylamino group by reaction with an alkali metal, phenyl or alkyl isocyanate, alkanoic acid anhydride or alkoxyoxalyl halide respectively. The reaction may be carried out at room temperature or under heating for a period of from 12 to 36 hours, and the product may be isolated by conventional separation, drying and crystallisation steps. If an alkoxyoxalyl halide is used, the reaction should be carried out in the presence of a base, preferably triethylamine, to bind the hydrogen halide formed.

All the above described processes are within the scope of the invention.

The pharmaceutically acceptable salts according to the invention may be prepared from the bases in a conventional manner. Preferred pharmaceutically acceptable acid addition salts are those of hydrochloric, sulphuric, nitric, phosphoric, acetic, propionic, succinic, maleic, citric, tartaric, benzoic, methane-sulphonic and toluenesulphonic acids.

The ω-[4-(2-pyridyl)-piperazino]-alkanoylanilides and their salts according to the invention possess valuable pharmacological properties, e.g. antianaphylactic, antibronchospastic, antihistaminic, sedative, analgesic, antiserotonic and blood-pressure-lowering effects.

Accordingly, the invention also provides pharmaceutical compositions comprising a compound of the general formula I as above defined or a pharmaceutically acceptable salt thereof in admixture with a pharmaceutically acceptable diluent or carrier.

The $LD_{50}$ of the alkanoylanilides according to the invention was determined in the mouse both i.p. and per os, following the method described by C.S.Weil (Biometrics, 8,249, 1952). The obtained results are reported here below.

TABLE 1

$LD_{50}$ in mM/Kg

| Active compound | ip | os |
|---|---|---|
| 1 | 0.90 | -- |
| 2 | 0.65 | -- |
| 3 | 0.71 | 1.16 |
| 4 | 0.42 | 2.07 |
| 5 | 0.91 | 1.21 |
| 6 | 0.47 | 0.81 |
| 7 | 1.11 | 1.29 |
| 8 | > 2.25 | > 6.7 |
| 11 | 1.05 | 1.53 |
| 12 | > 2.35 | > 7.05 |
| 13 | 0.48 | 1.5 |
| 14 | 3.25 | 5.97 |
| 15 | 0.57 | -- |
| 16 | 1.16 | 1.35 |
| 17 | 1.01 | 1.58 |
| 18 | 0.80 | -- |
| 19 | 0.69 | 0.92 |
| 20 | > 2.5 | 5.2 |

| Active compound | ip | os |
|---|---|---|
| 21 | 2.38 | 3.74 |
| 22 | 3.2 | 2.08 |
| 23 | 0.9 | 0.85 |
| 24 | 1.18 | 2.97 |
| 25 | 0.53 | 1.60 |
| 27 | -- | > 6.81 |
| 28 | > 2.15 | > 6.5 |
| 29 | 2.08 | 3.7 |
| 30 | 0.42 | 1.2 |
| 31 | -- | 4.39 |
| 32 | -- | 1.57 |
| 33 | 1.29 | 2.64 |
| 34 | -- | > 7.1 |
| 35 | 0.62 | 0.96 |
| 36 | 0.8 | 1.24 |
| 37 | 0.56 | 1.17 |
| 38 | 0.37 | 0.67 |
| 39 | 2.30 | > 6.91 |
| 40 | 0.68 | 1.11 |
| 41 | 0.66 | 0.92 |
| 42 | -- | > 8.4 |
| 43 | 0.39 | 0.62 |

In order to evaluate the antianaphylactic activity of the new compounds, the method of Goose and Blair was followed, in which passive cutaneous anaphylaxis was induced in the rat with homologous antibodies (Immunology, 16, 749, 1969). Female albino rats were immunized intramuscularly with egg albumin and intraperitoneally with Hemophylus pertussis vaccine.

Twelve days after the treatment, the animals were bled and sera thus obtained were injected intradermally into another group of rats.

Twenty-four hours later, the animals were challenged with an i.v. solution of ovoalbumin and Evans blue dye, and sacrificed after 30 minutes. The drugs to test were given by different routes of administration and at different times before the challenge.

Inhibition of spots areas ($ED_{50}$) was determined.

The obtained data are reported in table II.

TABLE II

PCA Test-$ED_{50}$ in mM/Kg

| Active compound | ip | os |
|---|---|---|
| 1 | 0.337 | -- |
| 2 | 0.212 | -- |
| 3 | 0.019 | |
| 4 | 0.056 | 0.068 |
| 5 | 0.089 | -- |
| 6 | 0.027 | -- |
| 7 | 0.027 | 0.074 |
| 11 | 0.16 | -- |
| 12 | 0.13 | 0.18 |
| 13 | 0.104 | -- |
| 14 | 0.032 | 0.106 |
| 15 | 0.019 | 0.026 |

| Active compound | ip | os |
|---|---|---|
| 16 | 0.061 | 0.064 |
| 17 | 0.067 | |
| 18 | 0.013 | 0.081 |
| 19 | 0.054 | -- |
| 20 | 0.17 | 0.12 |
| 21 | 0.16 | 0.27 |
| 22 | 0.1 | -- |
| 23 | 0.17 | -- |
| 24 | 0.039 | -- |
| 25 | 0.055 | 0.11 |
| 27 | 0.092 | 0.12 |
| 28 | 0.073 | -- |
| 30 | 0.23 | -- |
| 31 | 0.13 | -- |
| 33 | 0.047 | 0.151 |
| 35 | 0.039 | |
| 36 | 0.029 | -- |
| 38 | 0.044 | -- |
| 39 | 0.073 | |
| 40 | 0.073 | -- |
| 41 | 0.028 | 0.22 |

The antibronchospastic activity was performed according to the method of Konzett and Rössler (Arch. exp.Path. Pharmakol., 195, 71, 1940), determining the inhibition of bronchospasm induced in anaesthetized guinea-pig by histamine. For this purpose, the bronchospasms were induced administering intravenously to the animals

0.5-2.5 μg/Kg of hystamine. Inhibition ($ED_{75}$) was determined one minute after the injection of the drug under test.

TABLE III

$ED_{75}$ μM/Kg i.v.

| Active compound | Obtained values |
|---|---|
| 3 | 0.24 |
| 4 | 0.37 |
| 5 | 3.89 |
| 6 | 0.94 |
| 7 | 0.02 |
| 11 | 0.23 |
| 12 | 0.23 |
| 13 | 0.02 |
| 14 | 0.48 |
| 15 | 1.56 |
| 16 | 1.93 |
| 17 | 0.47 |
| 18 | 1.57 |
| 19 | 0.14 |
| 20 | 0.49 |
| 21 | 1.1 |
| 22 | 1.63 |
| 23 | 0.61 |
| 24 | 0.59 |
| 25 | 0.4 |
| 27 | 0.97 |
| 28 | 0.29 |
| 29 | 0.58 |

| Active Compound | Obtained Value |
|---|---|
| 30 | 1.75 |
| 32 | 2.4 |
| 33 | 1.12 |
| 35 | 2.27 |
| 36 | 0.4 |
| 39 | 0.89 |
| 40 | 0.2 |
| 41 | 0.17 |

The following Examples illustrate the invention.

EXAMPLE 1

2,6-Dimethyl-α-/4-(2-pyridyl)-piperazino/-acetanilide.
(I:R=$R_4$=$CH_3$,$R_1$=$R_2$=$R_3$=$R_5$=$R_6$=H,n=1). Active compound 1.

4.2 g of sodium bicarbonate and 9.88 g of 2,6-dimethyl-α-chloro-acetanilide dissolved in 100 ml of acetone were added to 8.16 g of 1-(2-pyridyl)-piperazine dissolved in 25 ml of acetone. The mixture was refluxed under stirring for 5 hours. When the reaction was over, the precipitate was filtered off, collected and washed with acetone. Active compound (1) was dried and crystallized from ethyl acetate.
Yield 8.8g, mp 144-145°C.
The free base, dissolved in isopropanol, was treated with hydrogen chloride in isopropanol to give the corresponding hydrochloride of mp 189-191°C.

EXAMPLE 2

2,3,5,6 - Tetramethyl-α-/4-(2-pyridyl)-piperazino/-acetanilide.
(I:R=$R_1$=$R_3$=$R_4$=$CH_3$,$R_2$=$R_5$=$R_6$=H, n=1). Active compound 2

To a mixture consisting of 1.63 g of 1-(2-pyridyl)-piperazine and 0.84 g of sodium bicarbonate in 5 ml of isopropanol, a suspension of 2.47 g of 2,3,5,6-tetramethyl-α-chloro-acetanilide in 30 ml of isopropanol was added at room temperature. The whole

was stirred and refluxed for 5 hours. When the reaction was over the crude product was filtered off, the solvent was evaporated off and the residue washed with boiling water, dried, and crystallized from ethyl acetate (or isopropanol). Yield 1.8 g, mp 151-152°C. The hydrochloride, obtained in the usual manner, melts 265-267°C.

EXAMPLE 3.

4-Methoxy-β-[4-(2-pyridyl)-piperazino]-propionylanilide. (I:$R=R_1=R_3=R_4=R_5=R_6=H$, $R_2=CH_3O$, $n=2$) Active compound 3.

To 3.19 g of 4-methoxy-acryloylanilide dissolved in 18 ml of benzene, 2.93g of 1-(2-pyridyl)-piperazine dissolved in 9 ml of benzene were added. The mixture was refluxed for 3 hours. When the reaction was over, the mixture was cooled, the precipate filtered off and crystallized from ethanol. Yield 4.52 g, mp 127-128°C.

The corresponding hydrochloride, crystallized from 95% ethanol as hemihydrate, melts 207-211°C.

EXAMPLE 4

3-Nitro-β-[4-(2-pyridyl)-piperazino]-propionylanilide (I:$R=R_2=R_3=R_4=R_5=R_6=H$, $R_1=NO_2$, $n=2$). Active compound 4.

13.45 g of 3-nitro-acryloylanilide (obtained from 3--nitroaniline and acrylic acid chloride) and 11.41 g of 1-(2-pyridyl)-piperazine in 140 ml of toluene were refluxed under stirring for 16 hours. At the end of the reaction, the mixture was cooled and the precipitate thus formed was filtered off and crystallized from toluene. Yield 22.19g., mp 133-134°C. The hydrochloride, crystallized from 90% ethanol, melts at 228-230°C. Starting from 4-nitro-acryloylanilide instead of 3-nitro-acryloylanilide, there was obtained active compound 6, 4-nitro-β-/4-(2-pyridyl)-piperazino/-propionylanilide (I:$R=R_1=R_3=R_4=R_5=R_6=H$, $R_2=NO_2$, $n=2$), mp 157-158°C.

EXAMPLE 5

2-Benzyloxy-5-acetyl-β-/4-(2-pyridyl)-piperazino/-propionylanilide.
(I:$R=R_2=R_3=R_5=R_6=H$, $R_1=COCH_3$, $R_4=C_6H_5CH_2O$, $n=2$).
Active compound 5.

A mixture comprising 14.75 g of 3-acryloylamino-4-benzyloxy-acetophenone and 8.1 g of 1-(2-pyridyl)-piperazine in 150 ml of dichloromethane was refluxed for 1 hour, and then allowed to stand for one day at room temperature. The solvent was evaporated off and the crude product thus formed purified on a silica gel column using ethyl acetate as eluent. Active compound 5 was crystallized from 60% ethanol or

ligroin, mp 134-135°C. Yield 14.5 g.

EXAMPLE 6

4-Amino-β-[4-(2-pyridyl)-piperazino]-propionylanilide. (I: $R=R_1=R_3=R_4=R_5=R_6=H, R_2=NH_2, n=2$). Active compound 7.

To 8.88 g of 4-nitro-β-[4-(2-pyridyl)-piperazino]-propionylanilide, obtained as described in Example 4, dissolved in 350 ml of methanol, 0.5 g of 10% palladium-on-carbon were added. The whole was hydrogenated at the initial pressure of 60lbs. After 8 hours the catalyst was collected on a filter and the methanol evaporated off. The residue was dissolved in chloroform and chromatographed on a silica gel column using a chloroform:methanol mixture (98:2 by volume) as eluent. The product was crystallized from ethyl acetate, yield 5.97 g, mp 103-104°C.

EXAMPLE 7

4-(3-Phenylureido)-β-[4-(2-pyridyl)-piperazino]-propionylanilide.
(I: $R=R_1=R_3=R_4=R_5=R_6=H, R_2=C_6H_5NHCONH, n=2$). Active compound 8

6.5 g of active compound 7, obtained as described in Example 6, and 2.38 g of phenylisocyanate in 60 ml of toluene were stirred at 20-25°C for 8 hours. After a night at room temperature, the solvent was

evaporated off under vacuum, and the residue was crystallized from dioxan:water (5:1 by volume) to give 6.09 g of the title compound Mp > 260°C.

## EXAMPLE 8

4-sulfamoyl-β-[4-(2-pyridyl)-piperazino]-propionylanilide ($I: R=R_1=R_3=R_4=R_5=R_6=H, R_2=NH_2SO_2, \underline{n}=2$). Active compound 9.

6.78g of 4- sulfamoylacryloylanilide and 5.38 g of 1-(2-pyridyl)-piperazine dissolved in 15 ml of DMSO, were heated at 50°C for 16 hours under stirring. At the end of the reaction, the mixture was poured in water and the product thus formed was collected, filtered off, dried, and crystallized from methanol. Yield 9g, mp 207-211°C.

## EXAMPLE 9

4-Ureido-β-[4-(2-pyridyl)-piperazino]-propionylanilide. ($I: R=R_1=R_3=R_4=R_5=R_6=H, R_2=NH_2\ CONH, \underline{n}=2$). Active compound 10.

To 3.25 g of active compound (7), obtained as described in Example 6, dissolved in 50 ml of 1N hydrochloric acid, 1.64 g of potassium isocyanate were added. The whole was stirred at 20 to 25°C for 8 hours. After a night at room temperature, the mixture was cooled

on ice and to it was added a saturated solution of sodium carbonate. The precipitate thus formed was filtered off, washed with hot acetone, filtered again and crystallized from methanol. Mp 250°C (with decomposition), yield 2.2 g.

EXAMPLE 10

4-Propionamido-β-[4-(2-pyridyl)-piperazino]-propionylanilide. ($I: R=R_1=R_3=R_4=R_5=R_6=H, R_2=C_2H_5CONH, n=2$). Active compound 11.

---

To 8.13 g of active compound 7, obtained as described in Example 6, in 35 ml of chloroform, 3.64 g of propionic anhydride were added. The mixture was refluxed under stirring for 8 hours. At the end of the reaction, the solvent was evaporated off under vacuum and the residue was treated with sodim bicarbonate. The product was filtered, dried, and crystallized from ethanol.

Yield 7.22 g, mp 195-197°C. The corresponding hydrochloride obtained as hydrate, melts at 190-192°C.

EXAMPLE 11

4-Ethoxyoxalylamido-β-[4-(2-pyridyl)-piperazino]-
-propionylanilide.
($I: R=R_1=R_3=R_4=R_5=R_6=H, R_2=C_2H_5OOC.CONH, n=2$).
Active compound 12.

To 8.13 g of active compound 7, obtained as described in Example 6, dissolved in 50 ml of chloroform, 4.2 ml of triethylamine and 3.35 ml of ethyl chloroxalate were added under stirring. Temperature was maintained

at $7^{\circ}C$ during the addition. After a night at room temperature, insoluble substances were removed from the solution, the solvent was evaporated off and the residue was suspended in water, filtered and crystallized from ethanol. Yield 6.5 g, mp 157-158°C.

EXAMPLE 12

4-Acetyl-β-[4-(2-pyridyl)-piperazinyl]-propionylanilide. ($I:R=R_1=R_3=R_4=R_5=R_6=H, R_2=CH_3CO, \underline{n}=2$). Active compound 13.

---

A mixture comprising 9.4 g of 4-acryloylamino-acetophenone and 8.1 g of 1-(2-pyridyl)-piperazine in 200 ml of benzene was maintained for 1 to 2 days at 20 to 25°C under stirring. When the reaction was over, the solvent was evaporated off and the residue chromatographed on a silica gel column using ethyl acetate:methanol (7:3 by volume) as eluent.

The crude product, after evaporation of the solvents, was crystallized from 40% ethanol to give 10 g of active compound 13, melting at 134-136°C. The corresponding hydrochloride, isolated as hemihydrate, melts at 235-240°C.

EXAMPLE 13

4-(1-Hydroxyethyl)-β-[4-(2-pyridyl)-piperazinyl]-propionylanilide.
($I:R=R_1=R_3=R_4=R_5=R_6=H, R_2=CH_3CH(OH), \underline{n}=2$. Active compound 14

---

To 14.8 g of active compound 13, obtained as described in Example 12, in 280 ml of ethanol, 1.52 g of sodium borohydride were added. The solution was maintained at 20 to 25°C for 27 hours. At the end of the reaction, the precipitate thus formed was separated off and suspended in water, and dilute hydrochloric acid was added until the pH reached 6. The mixture was then treated with sodium bicarbonate until the pH reached 8 and then the product was extracted with 500 ml of chloroform. The organic phase was separated off and gave a product crystallizing from ethanol. Yield 9.5 g, mp 157-161°C.

EXAMPLE 14

Following the procedures described in Examples 1 to 13, the compounds listed here below were prepared.

| ACTIVE COMPOUND | n | R | $R_1$ | $R_2$ | $R_3$ | $R_4$ | $R_5$ | $R_6$ | Mp$^{o}$C | |
|---|---|---|---|---|---|---|---|---|---|---|
| 15 | 2 | Me | Me | H | Me | Me | H | H | 176-8 | (176-81) |
| 16 | 2 | H | H | H | H | H | H | H | 79-81 | |
| 17 | 2 | Me | H | H | H | H | H | H | 99-102 | (201-2) |
| 18 | 2 | Me | H | H | H | Me | H | H | 121-2 | (134) |
| 19 | 2 | H | H | OEt | H | H | H | H | 129-30 | (228-9) |
| 20 | 2 | H | H | OH | H | H | H | H | 189-90 | (228-30) |
| 21 | 2 | H | $NO_2$ | OH | H | H | H | H | 148-9 | |
| 22 | 2 | $NH_2$ | H | H | H | H | H | H | 149-50 | |
| 23 | 2 | H | $NH_2$ | H | H | H | H | H | 148-50 | (230-1) |
| 24 | 2 | H | H | $NH_2$ | H | H | Me | H | 268-70 | |
| 25 | 2 | H | H | $NH_2$ | H | H | H | OMe | 96-8 | (207) |
| 26 | 2 | H | $NH_2$ | H | $NH_2$ | H | H | H | 155-6 | |
| 27 | 2 | H | H | NHCOMe | H | H | H | H | 187-8 | |
| 28 | 2 | H | H | NHCONHMe | H | H | H | H | 213-4 | (209-12) |
| 29 | 2 | $NO_2$ | H | H | H | H | H | H | 90-2 | (220-2) |

| ACTIVE COMPOUND | n | R | $R_1$ | $R_2$ | $R_3$ | $R_4$ | $R_5$ | $R_6$ | Mp°C | |
|---|---|---|---|---|---|---|---|---|---|---|
| 30 | 2 | H | H | $NO_2$ | H | H | Me | H | 129-30 | (210-2) |
| 31 | 2 | H | H | $NO_2$ | H | H | H | OMe | 126-8 | (207-11) |
| 32 | 2 | H | $NO_2$ | H | $NO_2$ | H | H | H | 186-7 | |
| 33 | 2 | Cl | H | H | H | H | H | H | 95-6 | (181-5) |
| 34 | 2 | $NO_2$ | H | H | H | H | H | OMe | 103-4 | (212-3) |
| 35 | 2 | H | H | Cl | H | H | H | H | 118-9 | (202-3) |
| 36 | 2 | Cl | H | H | H | Cl | H | H | 155-7 | (270 2) |
| 37 | 2 | H | $CF_3$ | H | H | H | H | H | 100-2 | (203-4) |
| 38 | 2 | $NH_2$ | H | H | H | H | H | OMe | 148-50 | (179 dec) |
| 39 | 2 | COMe | H | H | H | H | H | H | 90-1 | (230-2) |
| 40 | 2 | H | COMe | H | H | H | H | H | 109-11 | (223-5) |
| 41 | 2 | H | H | COOEt | H | H | H | H | 92-5 | (227-9) |
| 42 | 2 | H | H | SMe | H | H | H | H | 128-9 | |
| 43 | 2 | H | H | CN | H | H | H | H | 112-4 | (247-8) |
| 44 | 2 | H | H | $CONH_2$ | H | H | H | H | 227-8 | |
| 45 | 2 | H | OMe | H | H | H | H | H | 102-3 | |

When two melting points are reported, the second one refers to the salts.
If not differently indicated, the hydrochloride is intended.

EXAMPLE 15

3,6-Dimethyl-4-nitro-β-[4-(2-pyridyl)-piperazino]-propionylanilide. (I: $R=R_3=R_5=R_6=H, R_1=R_4=CH_3, R_2=NO_2$, n=2), Active compound 46.

Operating as described in Example 4 but starting from 3,6-dimethyl-4-nitro-acryloylanilide instead of 3-nitro-acryloylanilide, the title compound melting at 176-177°C was obtained.

This compound was transformed into its dihydrochloride which melted at 262-266°C. Using 10% palladium-on--carbon and hydrogenating, the corresponding 3,6-dimethyl-4-amino derivative was obtained, isolated as its trihydrochloride hydrate, mp 266-271°C (I: n=2, $R=R_3=R_5=R_6=H$, $R_1=R_4=CH_3$, $R_2=NH_2$ (Active compound 47). In the same manner, but starting from tetramethyl-4-nitro-acryloylanilide, 2,3,5,6- tetramethyl-4-nitro-β-[4-(2-pyridyl)-piperazino]--propionylanilide was obtained, melting at 166-167°C (the dihydrochloride melted at 273-275°C). From this compound, as described above, the corresponding amino derivative was prepared, mp 170-172°C. These compounds have the general formula I in which n = 2, $R_5=R_6=H$, $R=R_1=R_3=R_4=CH_3$, $R_2=NO_2$ or $NH_2$, and are Active compounds 48 and 49 respectively.

EXAMPLE 16

4-Sulphamoyl-β-[4-(2-pyridyl)-piperazino]-butyrylanilide. (I: $R=R_1=R_3=R_4=R_5=R_6=H$, $R_2= SO_2NH_2$, n=3). Active compound 50

To 17.2 g of 4-sulfamoylanilide in 8 ml of acetic acid, 12.3 ml of 4-chloro-butyryl chloride were added dropwise at room temperature and under stirring. After two hours, the homogenous paste thus obtained was treated with 33 g of sodium acetate dissolved in 150 ml of water. The whole was kept at room temperature for 12 hours. The solid form was then collected by filtration, washed with water, dried and crystallized from ethanol. Yield 10 g, mp 178-178.5°C. This intermediate i.e. 4-sulfanoyl-ω--chlorobutyrylanilide has never been described in literature before. To 2.76 g of 4-sulfamoyl-ω--chlorobutyrylanilide, obtained as above, dissolved in 1.5 ml of acetone, 1.06 g of sodium bicarbonate and 1.92 g of 4-(2-pyridyl)-piperazine dissolved in 10 ml of acetone were added. The mixture was refluxed for 16 hours . At the end of the reaction, the solid formed was collected by filtration, washed with chloroform, treated with hot 95% ethanol to remove the inorganic salt and then filtered. From concentrated mother liquors there crystallized a product which was collected by filtration and recrystallized from 95% ethanol. Yield 1.08 g of the title compound mp 217-219°C.

CLAIMS

1. An ω-[4-(2-pyridyl)-piperazino]-alkanoylanilide derivative of the general formula I

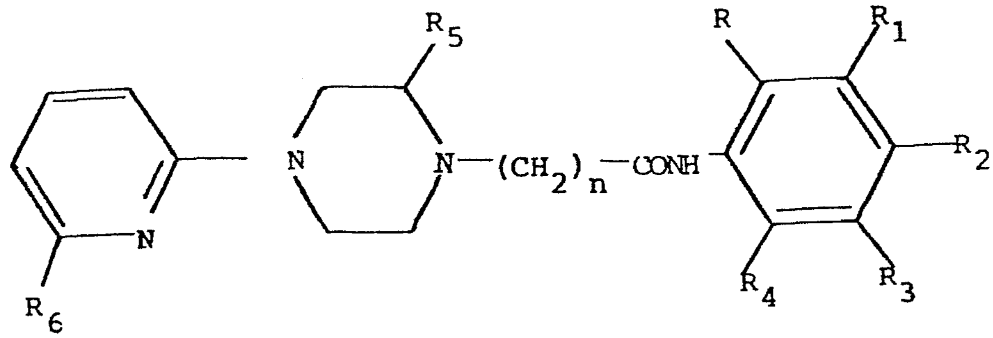

I

wherein n is 1,2, or 3, each of $R, R_1, R_2, R_3$ and $R_4$ independently represents a hydrogen or halogen atom, or an alkyl, alkoxy, hydroxy, nitro, amino, trifluoromethyl, cyano, alkylthio, 1-hydroxyalkyl, alkanoyl, carbamoyl, sulphamoyl, alkoxycarbonyl, alkylsulphonyl, benzyloxy, alkanoylamino, ureido, 3-phenylureido, 3-alkylureido or alkoxyoxalylamino group, $R_5$ represents a hydrogen atom or an alkyl group and $R_6$ represents a hydrogen atom or an alkoxy group, or a pharmaceutically acceptable acid addition salt of such an ω-[4-(2-pyridyl)-piperazino]-alkanoylanilide derivative.

2. 3-Nitro-β-[4-(2-pyridyl)-piperazino]-propionanilide.

3. 4-Nitro-β-[4-(2-pyridyl)-piperazino]-propionanilide.

4. 4-Amino-β-[4-(2-pyridyl)-piperazino]-propionanilide.

5. 4-Ethoxyoxalylamido-β-[4-(2-pyridyl)-piperazino]-propionanilide.

6. 4-Acetyl-β-[4-(2-pyridyl)-piperazino]-propionanilide.

7. 4-(1-Hydroxyethyl)-β-[4-(2-pyridyl)-piperazino]-propionanilide.

8. 2,3,5,6-Tetramethyl-β-[4-(2-pyridyl)-piperazino]-propionanilide.

9. β-[4-(2-Pyridyl)-piperazino]-propionanilide.

10. 2,6-Dimethyl-β-[4-(2-pyridyl)-piperazino]-propionanilide.

11. 4-Ethoxy-β-[4-(2-pyridyl)-piperazino]-propionanilide.

12. 4-Hydroxy-β-[4-(2-pyridyl)-piperazino]-propionanilide.

13. 4-Amino-β-[4-(6-methoxy-2-pyridyl)-piperazino]-propionanilide.

14. 4-(3-Methylureido)-β-[4-(2-pyridyl)-piperazino]-propionanilide.

15. 2-Chloro-β-[4-(2-pyridyl)-piperazino]-propionanilide.

Page 26a follows

16. 2-Amino-β-[4-(6-methoxy-2-pyridyl)-piperazino]-propionanilide.

17. 3-Acetyl-β-[4-(2-pyridyl)-piperazino]-propionanilide.

18. 4-Ethoxycarbonyl-β-[4-(2-pyridyl)-piperazino]-propionanilide.

19. 4-Methylthio-β-[4-(2-pyridyl)-piperazino]-propionanilide.

20. A process for the preparation of an ω-[4-(2-pyridyl)-piperazino]-alkanoylanilide derivative of the general formula I wherein n, $R_5$ and $R_6$ are as defined in claim 1 and each of R, $R_1$, $R_2$, $R_3$ and $R_4$ independently represents a hydrogen or halogen atom, or an alkyl, alkoxy, hydroxy, nitro, trifluoromethyl, cyano, alkylthio, 1-hydroxyalkyl, alkanoyl, carbamoyl, sulphamoyl, alkoxycarbonyl, alkylsulphonyl or benzyloxy group, the process comprising reacting a 4-(2-pyridyl)-piperazine of the general formula II

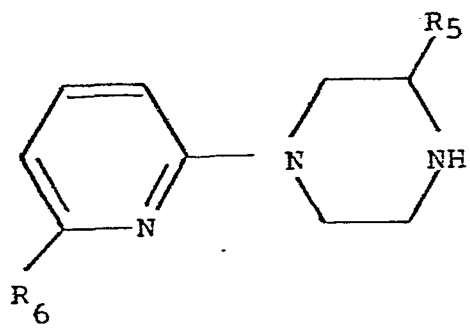

II

wherein $R_5$ and $R_6$ are as defined in claim 1 with an ω-halo-alkanoylanilide of the general formula III

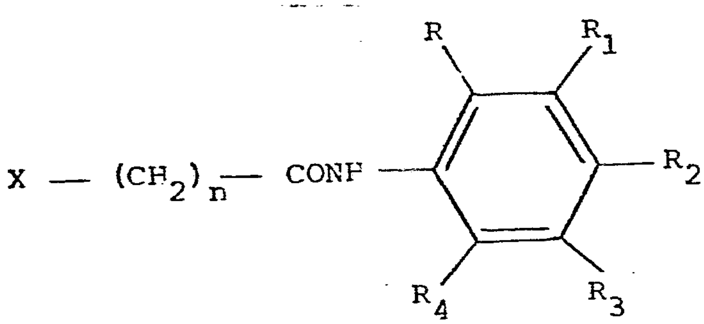

III

wherein X represents a halogen atom, n is as defined in claim 1 and $R,R_1,R_2,R_3$, and $R_4$ are defined in this claim.

21. A process according to claim 20 in which the reaction is carried out in the presence of an organic or inorganic base and a solvent, at the reflux temperature of the solvent.

22. A process for the preparation of an ω-[4-(2-pyridyl)--piperazino]-alkanoylanilide derivative of the general formula I wherein n is 2, $R_5$ and $R_6$ are as defined in claim 1 and $R,R_1,R_2,R_3$ and $R_4$ are as defined in claim 20, the process comprising reacting a 4-(2-pyridyl)--piperazine of the general formula II as defined in claim 20 with an acryloylanilide of the general formula IV

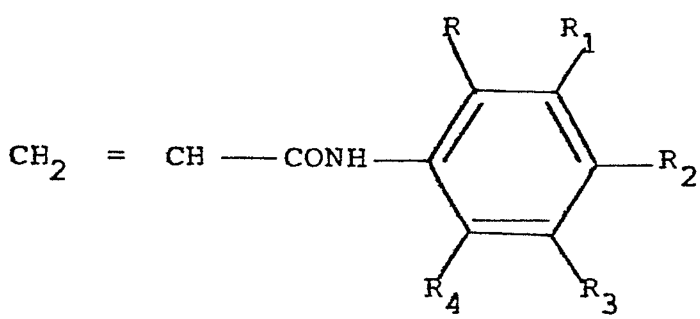

IV

wherein $R,R_1,R_2,R_3$ and $R_4$ are as defined in claim 20.

23. A process according to claim 22 in which the reaction is carried out in a solvent.

24. A process for the preparation of a compound of the general formula I as defined in claim 1 with the proviso that at least one of $R,R_1,R_2,R_3$ and $R_4$ represents

an amino group, the process comprising reducing, with hydrogen in the presence of a palladium-on-carbon catalyst and a solvent, a compound of the general formula I as defined in claim 1 with the proviso that the corresponding one(s) of $R, R_1, R_2, R_3$ and $R_4$ represent(s) a nitro group, the compound reduced having been obtained by a process according to claim 20 or claim 21.

25. A process for the preparation of a compound of the general formula I wherein n is 2 and $R, R_1, R_2, R_3, R_4, R_5$ and $R_6$ are as defined in claim 1 with the proviso that at least one of $R, R_1, R_2, R_3$ and $R_4$ represents an amino group, the process comprising reducing, with hydrogen in the presence of a palladium-on-carbon catalyst and a solvent, a compound of the general formula I wherein n is 2 and $R, R_1, R_2, R_3, R_4, R_5$ and $R_6$ are as defined in claim 1 with the proviso that corresponding one(s) of $R, R_1, R_2, R_3$ and $R_4$ represent(s) a nitro group, the compound reduced having been obtained by a process according to claim 22 or claim 23.

26. A process for the preparation of a compound of the general formula I as defined in claim 1 with the proviso that at least one of $R, R_1, R_2, R_3$ and $R_4$ represents a ureido, 3-phenylureido, 3-alkylureido, alkanoylamino or alkoxyoxalylamino group, the process comprising reacting a compound prepared according to claim 24 in which corresponding one(s) of $R, R_1, R_2, R_3$ and $R_4$ represent(s) an amino group with an alkali metal, phenyl

or alkyl isocyanate, an alkanoic anhydride or an alkoxyoxalyl halide.

27. A process for the preparation of a compound of the general formula I wherein n is 2 and $R, R_1, R_2, R_3, R_4, R_5$ and $R_6$ are as defined in claim 1 with the proviso that at least one of $R, R_1, R_2, R_3$ and $R_4$ represents a ureido, 3-phenylureido, 3-alkylureido, alkanoylamino or alkoxyoxalylamino group, the process comprising reacting a compound prepared according to claim 25 in which corresponding one(s) of $R, R_1, R_2, R_3$ and $R_4$ represent(s) an amino group with an alkali metal, phenyl or alkyl isocyanate, an alkanoic anhydride or an alkoxyoxalyl halide.

28. A pharmaceutical composition comprising an ω-[4-(2-pyridyl)-piperazino]-alkanoylanilide derivative according to any of claims 1 to 19 or a pharmaceutically acceptable salt thereof in admixture with a pharmaceutically acceptable diluent or carrier.